# EUROPÄISCHE PATENTSCHRIFT

(11) **EP 0 915 897 B1**
(45) Veröffentlichungstag und Bekanntmachung des Hinweises auf die Patenterteilung: **13.04.2005**
(21) Anmeldenummer: 97931663.5
(22) Anmeldetag: 24.06.1997
(51) Int. Cl.: C07H 1/06, C07H 21/00, C12Q 1/68

(54) **VERFAHREN ZUR KOMBINATION VON PARALLELER OLIGONUKLEOTIDSYNTHESE UND DARSTELLUNG VON OLIGOMER-CHIPS**
PROCESS FOR COMBINING PARALLEL OLIGONUCLEOTIDE SYNTHESIS AND PREPARATION OF OLIGOMER CHIPS
PROCEDE PERMETTANT DE COMBINER LA SYNTHESE PARALLELE D'OLIGONUCLEOTIDES ET LA PREPARATION DE COPEAUX D'OLIGOMERE

(30) Priorität: 25.06.1996 DE 19625397
(43) Veröffentlichungstag der Anmeldung: 19.05.1999
(73) Patentinhaber: Deutsches Krebsforschungszentrum Stiftung des öffentlichen Rechts, 69120 Heidelberg (DE)
(72) Erfinder: WEILER, Jan, D-69181 St. Ilgen (DE); HOHEISEL, Jörg, D-69168 Wiesloch (DE)
(74) Vertreter: Schüssler, Andrea, Dr.
(86) Internationale Anmeldenummer: PCT/DE1997/001332
(87) Internationale Veröffentlichungsnummer: WO 1997/049714

(56) Entgegenhaltungen:
- EP-A- 0 090 789
- WO-A-93/16118
- FR-A- 2 529 892
- S.IWAI ET AL.: "A NEW SOLID-PHASE SYNTHESIS OF OLIGORIBONUCLEOTIDES BY THE PHOSPHORO-P-ANISIDATE METHOD USING TETRAHYDROFURANYL PROTECTION OF 2'-HYDROXYL GROUPS" NUCLEIC ACIDS RESEARCH., Bd. 15, Nr. 9, 11.Mai 1987, OXFORD GB, Seiten 3761-3772, XP002047319
- PEASE A C ET AL: "LIGHT-GENERATED OLIGONUCLEOTIDE ARRAYS FOR RAPID DNA SEQUENCE ANALYSIS" PROCEEDINGS OF THE NATIONAL ACADEMY OF SCIENCES OF USA, Bd. 91, Nr. 11, 24.Mai 1994, Seiten 5022-5026, XP000569532 in der Anmeldung erwähnt
- KIERZEK R ET AL: "POLYMER-SUPPORTED RNA SYNTHESIS AND ITS APPLICATION TO TEST THE NEAREST-NEIGHBOR MODEL FOR DUPLEX STABILITY" BIOCHEMISTRY, Bd. 25, Nr. 24, 2.Dezember 1986, Seiten 7840-7846, XP000673484 in der Anmeldung erwähnt

## Beschreibung

Die Erfindung betrifft ein Verfahren zur Kombination von paralleler Oligonukleotidsynthese und Darstellung von Oligomer-Chips.

Angeregt durch die Kombination von Festphasentechnologie und der Phosphoramiditchemie, gab es große Fortschritte in der Automation der DNA-Synthese. Heute findet die Herstellung synthetischer Oligonukleotide, die für biologische, biomedizinische und physikalische Anwendungen gebraucht werden, in automatisierten DNA-Synthesegeräten statt. Diese Geräte produzieren Oligonukleotide im Nano- bis Mikromol-Bereich. Jedoch gab es in den letzten Jahren zwei gegenläufige Tendenzen bezüglich der Oligomersynthese. Für klinische Anwendungen, wie z.B. für die Antisense-Strategie, sind Gramm- oder sogar Kilogramm-Mengen an Oligonukleotiden erforderlich, was eine Erhöhung des Maßstabs bei der Oligomersynthese notwendig macht Dagegen sind für Anwendungen in der Molekularbiologie, z.B. bei der Polymerasekettenreaktion oder beim Sequenzieren von DNA, nur Oligomermengen im Picomolbereich erforderlich. Allerdings werden für diese Anwendungen eine Anzahl unterschiedlicher Oligonukleotide benötigt. Dies hat das Problem aufgeworfen, gleichzeitig verschiedene Oligomere in kleinen Mengen produzieren zu müssen. Andererseits spielt in der Molekularbiologie die sogenannte "Oligomer-Chip Technologie" eine zunehmende Rolle in der diagnostischen DNA-Analyse. Auch bezüglich vieler Methoden der Genomanalyse, z.B. für das "Sequenzieren durch Hybridisieren", steckt ein großes Potential in der Verwendung von Matrizen, auf die geordnete Oligonukleotid-Raster aufgebracht wurden.

Weiler et al. (Third International Symposium, Oxford 1993; Seiten 131-134) beschreiben eine "large-scale" Festphasensynthese nach der Phosphoramidit-Methode, wobei "beads" als Träger eingesetzt werden, die über eine Methylamino-modifizierte Oberfläche verfügen.

Die Aufgabe der vorliegenden Erfindung besteht deshalb darin, ein Verfahren bereitzustellen, mit dem es möglich ist, auf einfache Weise möglichst verschiedene Oligonukleotide bereitzustellen, die einerseits als "Oligomer-Chips" für Hybridisierungsexperimente dienen können, andererseits aber auch geordnet ablösbar und somit verfügbar für molekularbiologische Reaktionen wie PCR oder DNA-Sequenzierung sind.

Gelöst wird diese Aufgabe durch ein Verfahren gemäß Patentanspruch 1. Bevorzugte Ausführungsformen ergeben sich aus den Unteransprüchen.

Die Erfinder sind bei der Entwicklung ihres neuen Verfahrens von der "Oligomerchip-Technologie" (Southern, E.M. et al., Genomics 13, 1008-1017; Caviani-Pease, A.C. et al., Proc. Natl. Acad. Sci. U.S.A. 91, 5022-5026) ausgegangen, die bisher für die Sequenzierung von DNA eingesetzt wurde. Bei dieser Technik wird eine Matrix mit kurzen daran gebundenen Oligonukleotidsequenzen als Ziel für Hybridisierungsexperimente eingesetzt. Dabei wird darauf geachtet, daß die an die Matrix gebundenen Oligonukleotide fest damit verbunden sind.

Von den Erfindern wurde nun diese Bindung der Oligonukleotide an die Matrix so modifiziert, daß es einerseits möglich ist, den entstehenden Oligomerchip zu Hybridisierungsanalysen von DNA mittels Oligomerchip-Technologie einzusetzen oder die Oligonukleotide geordnet abzulösen, um sie dann für PCR-Zwecke oder bei der enzymatischen Sequenzierung von DNA bzw. als Sonde für Hybridisierungen einzusetzen.

Als Matrix, auf der die Oligonukleotide gebunden werden, kann eine Polymerfolie oder eine Glasoberfläche eingesetzt werden, bevorzugt ist jedoch eine aminierte Polypropylenfolie, die weiter oberflächenmodifiziert wird. Eine mögliche Oberflächenmodifizierung ist die Anbringung von Alkylamino-Gruppen, bevorzugt Methylamino-Gruppen. Dies geschieht beispielsweise dadurch, daß eine aminierte Polypropylenfolie in einem Gemisch von trockenem Dichlormethan, trockenem Dioxan, p-Nitrophenylchlorformiat und Triethylamin für einige Stunden geschüttelt wird. Nach Waschen mit Dichlormethan, wird die Folie in einem Gemisch von Pyridin und Acetanhydrid für einige Stunden geschüttelt, wodurch verbliebene Aminogruppen auf der Oberfläche abgesättigt werden. Nachfolgend wird die Folie mit Dichlormethan gewaschen und in Acetonitril oder Dimethylformamid aufgenommen. Es wird 1,6-Bis(methylamino)-hexan zugesetzt und das Gemisch für einige Stunden bis mehrere Tage geschüttelt. Nach Waschen mit DMF, Methanol und Aceton, wird die Methylamino-modifizierte Membran getrocknet.

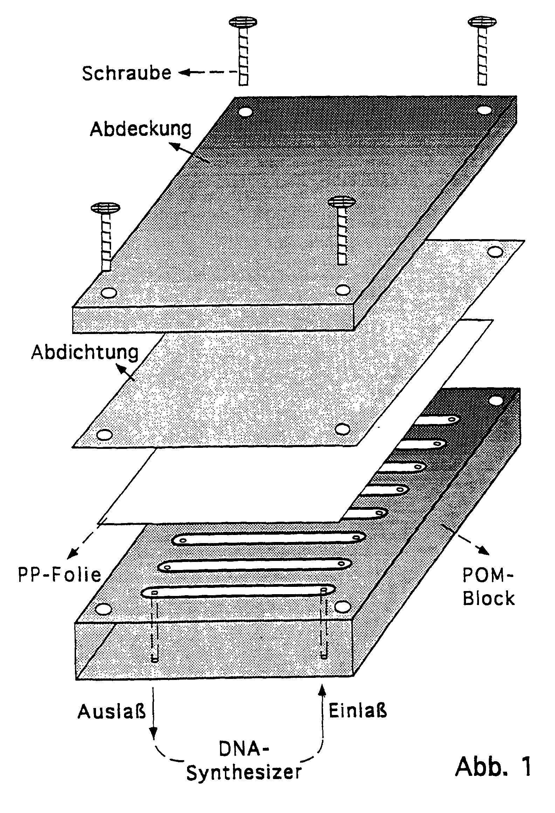

Eine vorstehende Matrix, insbesondere eine Methylamino-modifizierte Membran, kann in eine Synthesekammer,z.B. eine wie in Abb. 1 gezeigt, eingespannt werden. Auf die Matrix können 3'-Succinatderivate von geschützten Nukleosiden (dA^{NPEOC}, dC^{NPEOC}, dG^{NPEOC/NPE}, dT und Fluorescein-markiertes dC) aufgebracht werden, wobei die 3'-Succinatderivate wie in Kierzek et al. (Biochemistry 25, S. 7840-7846 (1986)) beschrieben, hergestellt werden können. Für das Aufbringen werden gewünschte Nukleosid-3'-Succinate mit einem organischen Lösungsmittel, z.B. N-Methylmorpholin und Acetonitril, gemischt, bevor O-[(Ethoxycarbonyl)cyanomethylenamino]N,N,N',N'-tetramethyluroniumtetrafluorborat (TOTU) zugesetzt wird. Dieses Reaktionsgemisch wird in die Synthesekammer gespeist und ein einfacher voreingegebener Zyklus in der Kammer aktiviert. Dieser Zyklus sieht beispielsweise so aus, daß die Reihen auf der Matrix, vorzugsweise der Methylamino-modifizierten Polypropylenfolie, mit dem Reaktionsgemisch befeuchtet werden und für eine bestimmte Zeit, z.B. 30 Minuten, inkubiert werden. Dann werden die Reagentien abgespült und die Reihen einige Male mit einem organischen Lösungsmittel, wie Acetonitril, gewaschen. Zum Blockieren der restlichen Methylaminogruppen auf der Matrix wird diese mit einem Gemisch von Pyridin und Acetanhydrid für mehrere Stunden behandelt. Alternativ kann die aus der Synthesekammer entfernte Matrix in einem Gemisch von Acetanhydrid, Pyridin und N-Methylimidazol behandelt werden. Nach Waschen mit DMF, Methanol und Aceton wird die Matrix getrocknet.

Für die Oligonukleotidsynthese kann es günstig sein, nachstehendes Schema zu verwenden. Zur Durchführung eignet sich z.B. die in Fig. 1 gezeigte Synthesekammer. Modifikationen des Schemas hinsichtlich Abfolge der Schritte, Austausch der Reagentien bzw. Lösungsmittel und Inkubationszeiten liegen im Können des Fachmanns und das Schema soll für die Erfindung in keinster Weise beschränkend ausgelegt werden.

**TABELLE 1**

| **Schema einer Oligonukleotidsynthese** | | |
|---|---|---|
| Schritt | Reagenz oder Lösungsmittel | Zeit (s) |
| Waschen | Acetonitril | 30 |
| | | |
| Detritylierung | 3 % TCA in Dichlormethan | 80 |
| | | |
| Waschen | Acetonitril | 210 |
| | | |
| Koppeln | 75 mM Phosphoramidit + 0,5 M Tetrazol in Acetonitril | 40 |
| | | |
| Waschen | Acetonitril | 30 |
| | | |
| Ablösen | Acetanhydrid/N-Methylimidazol in Acetonitril | 50 |
| | | |
| Oxidation | 1 M t-Butylhydroperoxid in Acetonitril | 130 |
| | | |
| Waschen | Acetonitril | 120 |

Nach vollständiger Synthese wird die Matrix aus der Synthesekammer entfernt und in 1 M 1,8-Diazabicyclo-(5.4.0)-undec-7en (DBU) in Acetonitril zur Deprotektion der Oligonukleotide geschüttelt. Vor dem Einsatz in Hybridisierungsexperimenten oder dem Ablösen der Oligomere wird die Matrix gewaschen, z.B. mit Acetonitril und Aceton.

Zum Ablösen der einzelnen Oligonukleotide wird die gewünschte Fläche des entstandenen Oligomerchips ausgeschnitten und nach einer Inkubation in 30% wässrigem Ammoniak für einige Stunden, z.B. 2 Stunden, werden die abgelösten Oligonukleotidprodukte lyophilisiert und für die PCR oder DNA-Sequenzierungsmethoden genutzt.

Bei der vorstehend beschriebenen NPE/NPEOC-Strategie werden die β-eliminierende Basenschutzgruppen 2-Nitrophenylethyl (NPE) und 2-(4-Nitrophenyl)-ethoxycarbonyl (NPEOC) verwendet. Diese Funktionen erlauben die Deprotektion des Biopolymers durch die starke, aber nicht-nukleophile Base DBU, während die Oligonukleotide auf der Matrix gebunden bleiben.

Die vorliegende Erfindung wird nun weiter mit Bezug auf die folgenden Abbildungen beschrieben:
- Abb. 1:: Reaktionskammer für die Oligonukleotidsynthese auf Polypropylenfolie, wobei das Gerät so ausgerichtet ist, daß es eine 90° Rotation der Polypropylenfolie zwischen den Syntheseschritten erlaubt.

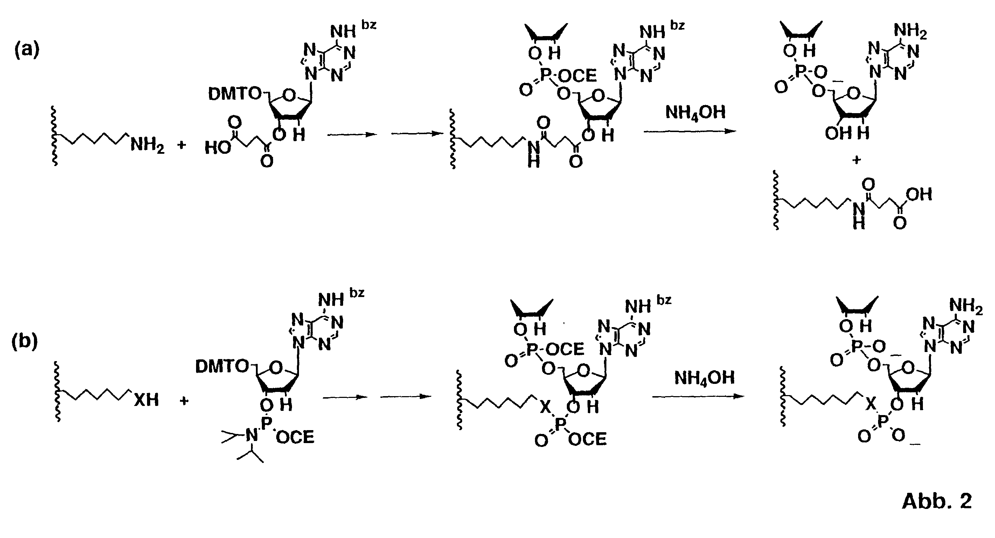

- Abb. 2:: Anbringung einer Probe und Ablösung durch Ammoniak (NH₄OH), verwendet für die Standardsynthese freier Oligonukleotide (a) und für die Herstellung von Oligonukleotid-Anordnungen (b); X = O; X = NH

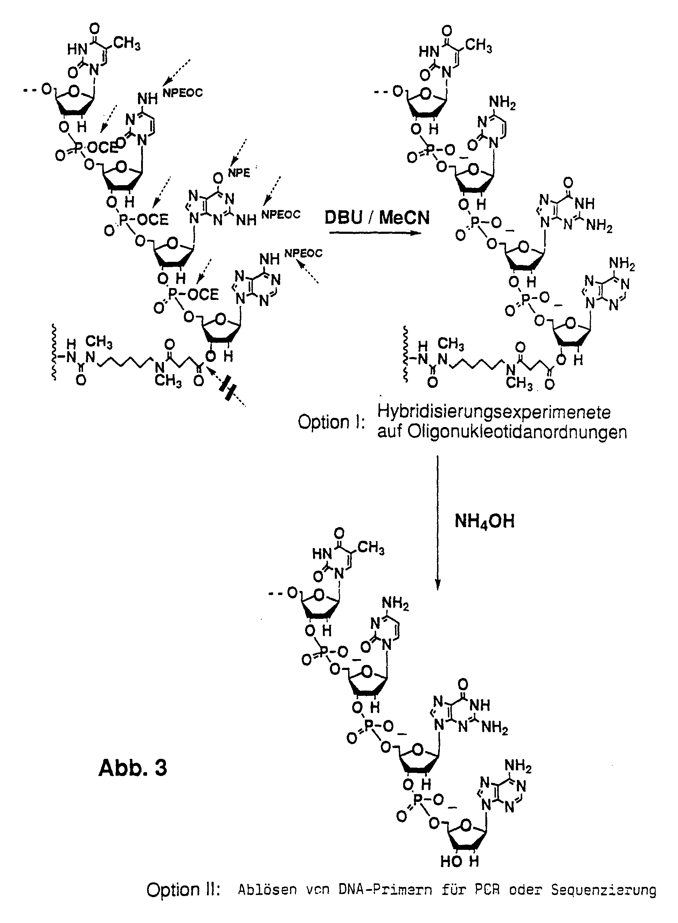

- Abb. 3:: Abtrennnung der Festphasen-gebundenen Oligonukleotide von der Oberfläche. Anwendung der "NPE/NPEOC-Strategie" erlaubt die alternative Verwendung der Oligomerchips für entweder Hybridisierungsexperimente (Option I) oder als Quelle für die Isolation einzelner Primermoleküle (Option II).

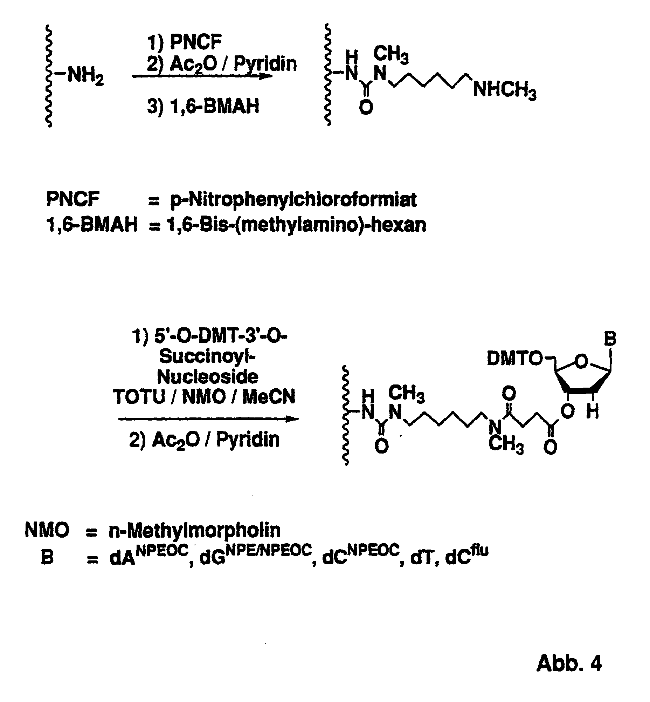

- Abb. 4:: Aktivierung der Methylamino-modifizierten Polypropylenfolie und Kopplung der geeignet geschützten Nukleoside.

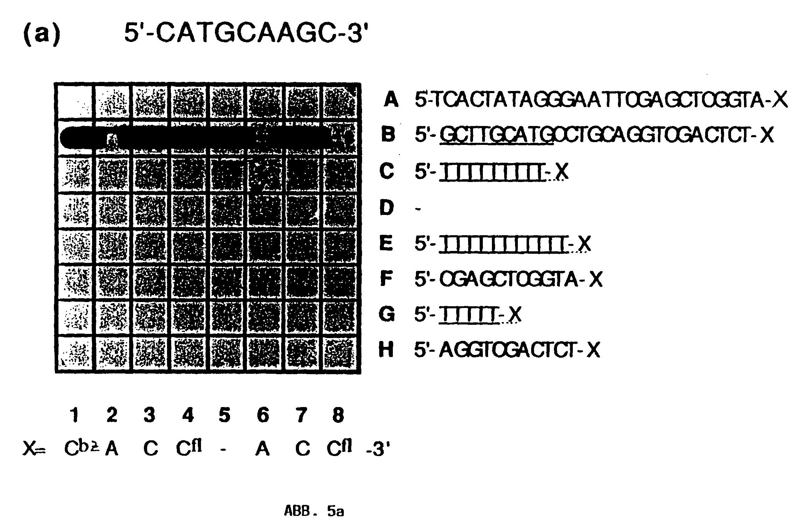

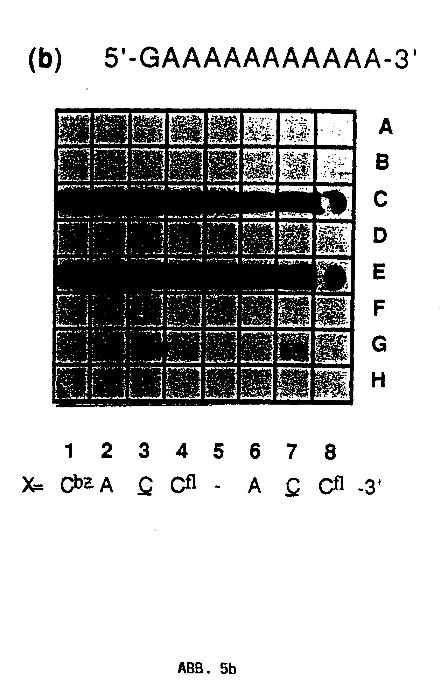

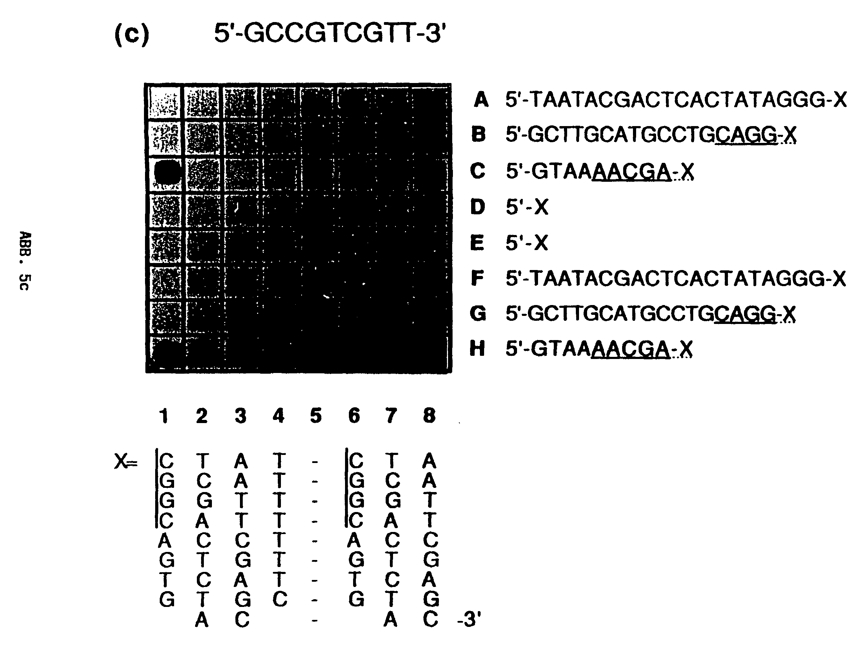

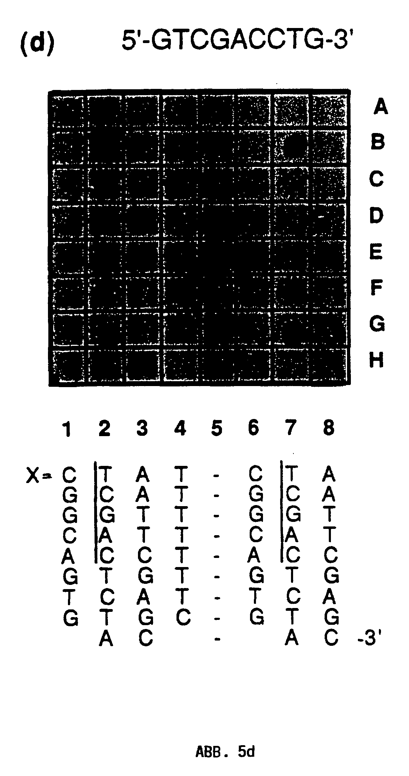

- Abb. 5:: Exemplarische Hybridisierungen zu Oligomeranordnungen auf Polypropylenmembranen. (a), (b): 4 verschiedene Startnukleoside, dC^{NPEOC} (Säulen 3,7), dA^{NPEOC} (Säulen 2,6), dC^{bz} (Säule 1) und dC^{f1} (Säulen 4,8) wurden an eine Polypropylenfolie unter Verwendung eines 8-Kanal-Synthesegeräts aufgebracht. Die Folie wurde dann aus der Reaktionskammer entfernt und um 90° gedreht. Nachfolgend wurden 7 verschiedene Oligonukleotide synthetisiert (A-H), die 49 Plätze mit 28 verschiedenen Sequenzen erzeugen. Ein Kanal wurde in beiden Dimensionen als Kontrolle leer gelassen (Spuren 5 und D). Nach Oligomerdeprotektion wurden einige Plätze (z.B. B2,6,8; C8; E8) zur Analyse ausgeschnitten. Die verbleibende Folie wurde mit einem (a) 9-mer d(CTATAGTGA) und (b) einem 12-mer d(GA₁₁) hybridisiert. Komplementäre Sequenzen sind unterstrichen. In (c) und (d) wurde die Folie zwischen den Syntheseschritten um 90° gedreht. Es wurden Nonamere hybridisiert, deren Sequenzen Bruchpunkte der Synthese abdecken.
- Abb. 6:: PCR-Amplifikation eines Plasmid-Inserts. Beide kommerziellen Primer wurden für die Reaktion verwendet, die in Spur 1 aufgetragen ist. In den Spuren B und C wurde jeder der Primer durch Oligonukleotide ersetzt, die von einem 0,16 cm² Stück der erfindungsgemäß behandelten Polypropylenfolie isoliert worden sind. Marker (Spur D) ist HindIII-verdaute λ-DNA und mit Fspl geschnittene Plasmid pUC18-DNA.

### BEISPIEL

Ein Polyoxymethylen (POM)-Block wurde so hergestellt, daß er 8 Kanäle von jeweils 1 mm Tiefe, 70 mm Länge und 4 mm Breite mit 2 Löchern an beiden Enden für den Anschluß an den Einlaß und Auslaß eines üblichen DNA-Synthesegeräts enthält. Der Polypropylenfilm wurde durch eine Silikonabdichtung und einen Plexiglasdeckel, der auf die POM-Basis geschraubt ist, an Ort und Stelle gehalten (Fig. 1). Um eine perfekte Abdichtung zu erhalten, wurde auf das gesamte Gerät mittels einer Klammer zusätzlicher Druck angewendet. Durch Entfernen der Polypropylenfolie nach einem einzelnen oder mehreren Zyklen, Rotation um 90° und fortgesetzte Synthese erlaubt dieses einfache und kleine instrument die Synthese von bis zu 64 verschiedenen Oligomeren. Eine aminierte Polypropylenfolie (8 x 8 cm²) wurde in einem Gemisch von 25 ml trockenem Dichlormethan, 25 ml trockenem Dioxan, 0,2 g p-Nitrophenylchlorformiat und 160 µl Triethylamin für 2 h bei Raumtemperatur leicht geschüttelt. Nach Waschen mit Dichlormethan wurde die Folie in einem 1:1-Gemisch von Pyridin und Essigsäureanhydrid für 2 h geschüttelt, wodurch die auf der Oberfläche verbliebenen Aminogruppen abgesättigt wurden. Nachfolgend wurde die Folie mit Dichlormethan gewaschen und in 50 ml Acetonitril oder Dimethylformamid (DMF) aufgenommen; es wurden 0,2 ml 1,6-Bis-(methylamino)-hexan hinzugefügt und das Gemisch wurde bei 40°C für 48 h geschüttelt. Nach aufeinanderfolgendem Waschen mit DMF, Methanol und Aceton wurde die Methylamino-modifizierte Propylenfolie getrocknet und bei 4°C aufbewahrt.

3'-Succinatderivate von geschützten Nukleosiden (dA^{NPEOC}, dC^{NPEOC}, dG^{NPEOC/NPE}, dT und Fluorescein-markiertes dC (= dC^{fl}) wurden durch das von Kierzek et al. (Biochemistry 25, Seiten 7840-7846 (1986)) beschriebene Verfahren hergestellt und auf einer Methylamino-modifizierten Polypropylenfolie, nach Befestigen der Folie in dem Polyoxymethylen-Block, aufgebracht. Dazu wurden 5 mg des gewünschten Nukleosid-3'-Succinats mit 25 µl N-Methylmorpholin und 10 ml Acetonitril vor Zugabe von 4 mg TOTU gemischt. Die Flasche mit dem Gemisch wurde sofort mit einem DNA-Synthesegerät verbunden und ein einfacher vorprogrammierter Zyklus in dem Gerät wurde aktiviert: zuerst wurden die Reihen mit dem Reaktionsgemisch benetzt und die Reaktion wurde 30 min inkubiert; dann wurden die Reagenzien mit Argon fortgespült und die Reihen wurden einige Male mit Acetonitril gewaschen. Um restliche Methylamino-Gruppen auf der Polypropylenfolie zu blockieren, wurde Essigsäureanhydrid und N-Methylimidazol in Acetonitril aufgebracht. Alternativ wurden die derivatisierten Polypropylenmembranen aus der Kammer entfernt und in einem Gemisch von 10 ml Essigsäureanhydrid, 10 ml trockenem Pyridin und 1 ml N-Methylimidazol für 2 h in einer Polypropylenbox geschüttelt. Nach nachfolgendem Waschen mit DMF, Methanol und Aceton wurden die Folien getrocknet und bis zur Verwendung bei 4°C aufbewahrt.

Die Oligonukleotidsynthese wurde auf der Polypropylenfolie wie in der vorstehenden Tabelle 1 ersichtlich ausgeführt. Nach kompletter Synthese wurden die Folien aus der Kammer entfernt und in 1 M-DBU in Acetonitril in einer Polypropylenbox bei 40°C über Nacht geschüttelt. Die Membran wurde mit Acetonitril und Aceton gewaschen, bevor sie entweder für Hybridisierungsexperimente oder zum Ablösen der Oligomere eingesetzt wurde.

Oligonukleotidsonden wurden mit [γ³²P] ATP unter Standardbedingungen endmarkiert. Die Oligomeranordnungen wurden in 600 mM NaCl, 60 mM Natriumcitrat, pH 7,5, 7,2% Natrium-N-Lauroylsacrosin für 1 h prähybridisiert und dann in 10 ml der selben Lösung, die ungefähr 1 Mcpm radioaktiv markierte Oligomersonde (Konzentration = 6 picomol/ml) enthielt, für 18 h bei 4°C inkubiert. Nach 30minütigem Waschen bei 4°C wurde eine Autoradiographie bei -70°C durchgeführt. Die Sonden wurden von den Folien durch inkubation in Hybridisierungspuffer bei 65°C für 3 h abgelöst. Die Ergebnisse der Hybridisierungen sind in Abb. 5, insbesondere den Abb. 5A und 5B, gezeigt.

Zum Ablösen der einzelnen Oligonukleotide wurden die gewünschten Plätze der Oligomeranordnung auf der Polypropylenfolie ausgeschnitten. Nach Inkubation in 30% wäßrigem Ammoniak für 2 h wurden die abgelösten Oligonukleotidprodukte lyophilisiert und für die PCR und DNA-Sequenzierungsexperimente ohne weitere Reinigung eingesetzt.

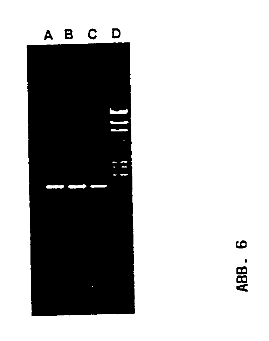

Um die Eignung der Oligomere für die PCR zu testen, wurde eine PCR bei dem rekombinanten Plasmid pTZ18R unter Standardbedingungen, wie bereits früher beschrieben (Scholler et al., Nucleic Acids Res. 23, Seiten 3842-3849, (1995)) durchgeführt. Primer waren 26- und 29-Mere, die an den Vektor direkt neben jeder Seite der Insert-DNA binden. Eine Oligonukleotidmenge, die einer Fläche von 0,16 cm² der Polypropylenoberfläche entsprach, wurde in den Reaktionen mit 25 µl Volumen verwendet. Eine PCR wurde ausgeführt: Primer-Annealing und Extension bei 68 ° C, Strangdenaturierung bei 95°C. Auf einem Agarosegel wurden die Produkte mit den Ergebnissen verglichen, die mit üblichen kommerziellen Primermolekülen erhalten wurden, die in einer Konzentration von 1 µM eingesetzt worden waren. Wie aus Abb. 6 ersichtlich, ergab sich kein signifikanter Unterschied bezüglich Qualität und Quantität der PCR-Produkte.

Auch die Verwendung in einer enzymatischen Sequenzierungsreaktion zeigte, daß die erfindungsgemäß erhaltenen und abgelösten Oligomere gegenüber käuflichen keine signifikanten Qualitätsunterschiede zeigen.

## Patentansprüche

1. Verfahren zur parallelen Synthese von Oligonukleotiden auf einer Alkylamino-modifizierten Matrixoberfläche, **dadurch gekennzeichnet, daß** 3'-Succinat-Derivate von geschützten Nukleosiden darauf gereiht angebracht werden und Oligonukleotidsynthese mittels automatisierter DNA-Synthese stattfindet.

2. Verfahren nach Anspruch 1, wobei die Matrixoberfläche mit Methylamino-Gruppen modifiziert ist.

3. Verfahren nach Anspruch 1 oder 2, wobei die 3'-Succinat-Derivate von dA^{NPEOC}, dC^{NPEOC}, dG^{NPEOC/NPE}, dT und Fluorescein-markiertem dC sind.

4. Verfahren nach einem der Ansprüche 1 bis 3, wobei die Matrix in einer Synthesekammer mit mehreren Kanälen eingespannt ist.

5. Verfahren nach einem der Ansprüche 1 bis 4, wobei die Matrix aus Glas oder einem Polymer, bevorzugt Polypropylen, ist.

6. Verwendung des bei dem Verfahren nach einem der Ansprüche 1 bis 5 entstehenden Oligomer-Chips, die die in Anspruch 1 angegebene Matrixoberfläche haben, in Hybridisierungsexperimente oder als Quelle hochqualitativer Biopolymere.

## Claims

1. A process for the parallel synthesis of oligonucleotides on an alkylamino-modified matrix surface, **characterised in that** 3'-succinate derivatives of protected nucleosides are attached thereto in rows and oligonucleotide synthesis takes place by means of automated DNA synthesis.

2. The process according to claim 1, wherein the matrix surface is modified with methylamino groups.

3. The process according to claim 1 or 2, wherein the 3'-succinate derivatives are from dA^{NPEOC}, dC^{NPEOC}, dG^{NPEOC/NPE}, dT and fluorescein-labeled cD.

4. The process according to any one of the claims 1 to 3, wherein the matrix is fixed in a synthesis chamber having several channels.

5. The process according to any one of the claims I to 4, wherein the matrix is made of glass or a polymer, preferably polypropylene.

6. Use of the oligomer chips forming in the process according to any one of the claims 1 to 5 and having the matrix surface as defined in claim 1 in hybridization experiments or as a source of high-quality biopolymers.

## Revendications

1. Procédé pour la synthèse parallèle d'oligonucléotides sur une surface de matrice modifiée par alkylamino, **caractérisé en ce que** des dérivés 3'-succiniques de nucléosides protégés y sont disposés en rangée et **en ce que** la synthèse des oligo-nucléotides s'effectue au moyen d'une synthèse ADN automatisée.

2. Procédé selon la revendication 1, dans lequel la surface de matrice est modifiée par des groupes méthytamino.

3. Procédé selon la revendication 1 ou 2, dans lequel les dérivés 3'-succiniques sont dA^{NPEOC}, dC^{NPEOC}, dG^{NPEOC/NPE}, dT et dC marquée à la fluorescéine.

4. Procédé selon l'une quelconque des revendications 1 à 3, dans lequel la matrice est insérée dans une chambre de synthèse à canaux multiples.

5. Procédé selon l'une quelconque des revendications 1 à 4, dans lequel la matrice est formée de verre ou d'un polymère, de préférence du polypropylène.

6. Application de puces oligomères obtenues à l'aide du procédé selon l'une quelconque des revendications 1 à 5, lesquels présentent la surface des matrices telles que décrites dans la revendication 1 dans des expériences d'hybridation ou en tant que source de biopolymère de haute qualité.
